Europäisches Patentamt

European Patent Office

Office européen des brevets

(19)

(11) EP 0 677 733 B1

(12) EUROPÄISCHE PATENTSCHRIFT

(45) Veröffentlichungstag und Bekanntmachung des Hinweises auf die Patenterteilung:
11.04.2001 Patentblatt 2001/15

(51) Int Cl.[7]: **G01N 21/39**, H01S 3/08, H01S 3/1055

(21) Anmeldenummer: 95100163.5

(22) Anmeldetag: 07.01.1995

(54) **Gaslaser und Gasnachweis damit**

Gas laser and detection of a gas therewith

Laser à gaz et détection de gaz avec celui-ci

(84) Benannte Vertragsstaaten:
**CH DE DK FR GB IT LI SE**

(30) Priorität: 25.01.1994 DE 4402054

(43) Veröffentlichungstag der Anmeldung:
18.10.1995 Patentblatt 1995/42

(73) Patentinhaber:
• **Carl Zeiss**
**89518 Heidenheim (Brenz) (DE)**
Benannte Vertragsstaaten:
**CH DE DK FR IT LI SE**
• **CARL-ZEISS-STIFTUNG,**
**HANDELND ALS CARL ZEISS**
**89518 Heidenheim (Brenz) (DE)**
Benannte Vertragsstaaten:
**GB**

(72) Erfinder:
• **Rupp, Wolfgang, Dr.**
**D-73434 Aalen (DE)**
• **Sauer, Ralf-Roland**
**D-73460 Hüttlingen (DE)**
• **Hinz, Alexander, Dr.**
**D-89551 Königsbronn (DE)**

(56) Entgegenhaltungen:
**EP-A- 0 196 856** **US-A- 5 002 391**

• **SOVIET JOURNAL OF QUANTUM ELECTRONICS, Bd.12, Nr.1, Januar 1982, NEW YORK US Seiten 110 - 112 A.G.GERASIMCHUK ET AL. 'Frequency-switching waveguide CO2 laser for atmospheric pollution monitoring'**
• **APPLIED PHYSICS, Bd.B38, 1985, HEIDELBERG, DE Seiten 37 - 40 K.UEHARA 'Alternate Intensity Modulation of a Dual-Wavelength He-Ne Laser for Differential Absorption Measurements'**
• **W. J. WITTEMAN: "The CO2 Laser", 1987, SPRINGER, BERLIN**

## Beschreibung

Die Erfindung betrifft einen Gaslaser, der durch sinusförmige Verschiebung eines Resonatorspiegels oder eines Gitters bei zwei verschiedenen Wellenlängen moduliert emittiert. Sie betrifft auch seine Verwendung in einer Anordnung zum Stoffnachweis durch unterschiedliche Absorption von Licht zweier Wellenlängen, sowie eine entsprechende Anordnung zum Stoffnachweis.

Aus EP-A 0 196 856 und Appl. Phys. B, 38, 37-40 (1985) ist ein gattungsgemäßer Laser zum Gasnachweis bekannt, der mit zwei Emissionswellenlängen arbeitet und durch Sinusschwingungen eines piezoelektrisch angetriebenen Resonatorspiegel moduliert wird. Es handelt sich um einen IR-He-Ne-Laser mit Methanzelle. Die Modulation erfolgt mit 1kHz derart, daß die Summe der Intensitäten bei beiden Wellenlängen zusammen weitgehend konstant bleibt. Zum Nachweis wird nichts weiter angegeben.

In A.G. Gerasimchuk et al., Sov. J. Quantum Electron 12 (1), (Jan. 1982), p. 110-112 wird der Ammoniak-Nachweis mit einem zwei-Wellenlängen-$CO_2$-Laser beschrieben. Die Wellenlängen-Modulation erfolgt durch einen piezoelektrisch bewegten Spiegel mit Rechteck-Steuerpulsen. Es ist ein Synchrondetektor vorgesehen.

In A. Stein et al., Applied Optics, Vol. 22 No. 21, (Nov. 1983) p 3378-3381, US 4 471 220 (Exxon) und US 5 002 391 (Mütek) wird der Nachweis von $NH_3$ in Rauchgas von Feuerungsanlagen mit zwei Wellenlängen von isotopenselektierten $^{13}CO_2$-Lasern beschrieben. Stein und US 4 471 220 verwenden zwei getrennte Laser für die beiden Wellenlängen und zwei mechanische Chopper. US 5 002 391 hat einen einzigen Laser, dessen Spiegel oder Gitter piezoelektrisch mit Rechteckpulsen verstellt wird, um abwechselnd je eine der beiden Wellenlängen zu erzeugen. Ein synchronisierter mechanischer Chopper bewirkt eine Aus-Tastung nach jedem Puls-Paar bei beiden Wellenlängen. Zum Nachweis dienen pyroelektrische Detektoren und sample-and-hold-Schaltungen.

GB-A 2 127 537 beschreibt einen Gasdetektor mit einem Laser, vorzugsweise $CO_2$-Laser ohne Gitter, der durch einen piezoelektrisch verstellten Resonatorspiegel bei verschiedenen Wellenlängen emittieren kann. Im Meßstrahlengang ist ein mechanischer Chopper, ein gekühlter Photodetektor und eine phasensensitive Detektorschaltung vorgesehen. Eine Referenzzelle mit pyroelektrischem Detektor ist obligatorisch.

W.J. Witteman, "The $CO_2$ Laser", Springer, 1987, S. 117-118 offenbart einen $CO_2$-Laser, dessen Resonatorlänge sägezahnförmig mittels eines piezoelektrischen Verschiebeelements moduliert wird, wobei zwei benachbarte Rotationsübergänge einer Vibrationsmode im Emissionsspektrum des Lasers voneinander getrennt sind und während einer stetigen Änderung der Rampenspannung am Verschiebeelement abwechselud im Emissionsspektrum auftauchen.

Aufgabe der Erfindung ist die Bereitstellung eines gattungsgemäßen Gaslasers, der ohne externe Schaltmittel, z.B. Chopper, im Nachweisstrahlengang als Lichtquelle für den Zwei-Wellenlängen-Absorptions-Stoffnachweis geeignet ist bei einfachem, robustem Aufbau und elektronisch gut verarbeitbarer Signalcharakteristik. Es ist auch die Angabe eines Gesamtsystems für den Laser-Stoffnachweis gefordert, in dem diese Lasereigenschaften optimal genutzt werden.

Gelöst wird diese Aufgabe dadurch, daß bei einem gattungsgemäßen Gaslaser die Dimensionierung eines Abstimmgitters hinsichtlich Gitterstruktur und Winkellage, des Auskoppelgrads des Auskoppelspiegels, der Resonatorlänge und der Gaszusammensetzung derart gewählt wird, daß der Laser in zwei nebeneinanderliegenden Intervallen der Resonatorlänge bei den zwei gewünschten Wellenlängen emittiert und am Übergang zwischen den zwei Intervallen die Laseremission unterbunden ist, und daß die sinusförmige Modulation der Resonatorlänge im wesentlichen symmetrisch um diesen Übergang liegt.

Vorteilhafte Ausführungsformen sind Gegenstand der Unteransprüche 2 - 5.

Die Verwendung eines derartigen Gaslasers in einer Anordnung zum Stoffnachweis, besonders für den Spurengasnachweis in Gas, und da besonders für den Ammoniaknachweis in Rauchgas, bringt die gesuchten Vorteile einer solchen Anordnung.

Eine erfindungsgemäße Anordnung nach Anspruch 8 sieht zusammen mit an sich bekannten Komponenten den Einsatz eines Gaslasers nach einem der vorherigen Ansprüche vor. Vorteilhafte Ausführungen gemäß den folgenden Ansprüchen betreffen die offene Ausführung der Meßstrecke mit einem Reflektor; eine Referenzstrecke für Eichzwecke mit einer Zelle; die Anordnung der Meßstrecke in einem Rauchgaskanal, den Nachweis von $NH_3$; Mittel, z.B. austauschbare oder einschwenkbare Dämpfungsfilter, zur Anpassung der Laserleistung an die Stoffkonzentration bzw. an die absorbierende und streuende Wirkung von Staub, Rauch und andere Untergrundeffekte - und damit zur Erhöhung der Sensordynamik.

Gemäß Anspruch 13 ist es besonders vorteilhaft, daß eine erfindungsgemäße Anordnung ohne Chopper bzw. ohne einen dritten Detektor für einen Referenzstrahl durch eine kalibrierte Zelle auskommt. Der apparative Aufwand ist dadurch deutlich vermindert und die Betriebssicherheit ist erhöht. Als Meßdetektor wird ein MCT-Detektor vorgezogen. Der bevorzugte Aufbau der Auswerteeinrichtung sieht AC-Kopplung des Referenz- und des Meßdetektors, eine Verstärkungschaltung und eine Integrationseinrichtung vor, sowie einen Analog-Digital-Wandler und einen programmierbaren Rechner.

Dabei ist es bevorzugt, daß die Integrationseinrichtung das vom Detektor aufgenommene Signal jeweils über den überwiegenden Teil des Zeitintervalls, in dem der Gaslaser bei einer der beiden Wellenlängen emittiert, aufintegriert. Damit kann ein sehr rauscharmes Nutzsignal sehr schnell gewonnen werden. Möglich ist dies vor allem durch die Sinusmodulation, die störungsarme Signalverläufe ergibt. Zur Erhöhung der Sensordynamik bei günstiger Auslegung der weiteren elektronischen Schaltungskomponenten kann die Verstärkungsschaltung in ihrem Verstärkungsfaktor abhängig vom Signal des Meßdetektors automatisch verstellt werden, z.B. um die Faktoren 10, 100 und 1000.

Ein weiterer Vorteil ist in Anspruch 19 niedergelegt. Es ist nämlich nur durch geeignete Programmierung des Rechners zugleich möglich, die Rauchdichte im Abgas zu bestimmen, und zwar aus der Transmission bei der Referenzwellenlänge Lambda 2, die nicht von Ammoniak beeinflußt ist.

Näher erläutert wird die Erfindung an einem Ausführungsbeispiel mit Hilfe der Zeichnung.

Darin zeigt:

Fig. 1      schematisch den Aufbau eines erfindungsgemäßen Lasers (Beispiel);

Fig. 2      die Signatur eines erfindungsgemäßen Lasers;

Fig. 3      ein schematisches Beispiel für den optischen Aufbau einer Anordnung zum Stoffnachweis;

Fig. 4      den Verlauf des Signals am Meßdetektor einer Anordnung nach Fig. 3;

Fig. 5      ein Blockschaltbild einer Schaltung für eine erfindungsgemäße Anordnung zum Stoffnachweis.

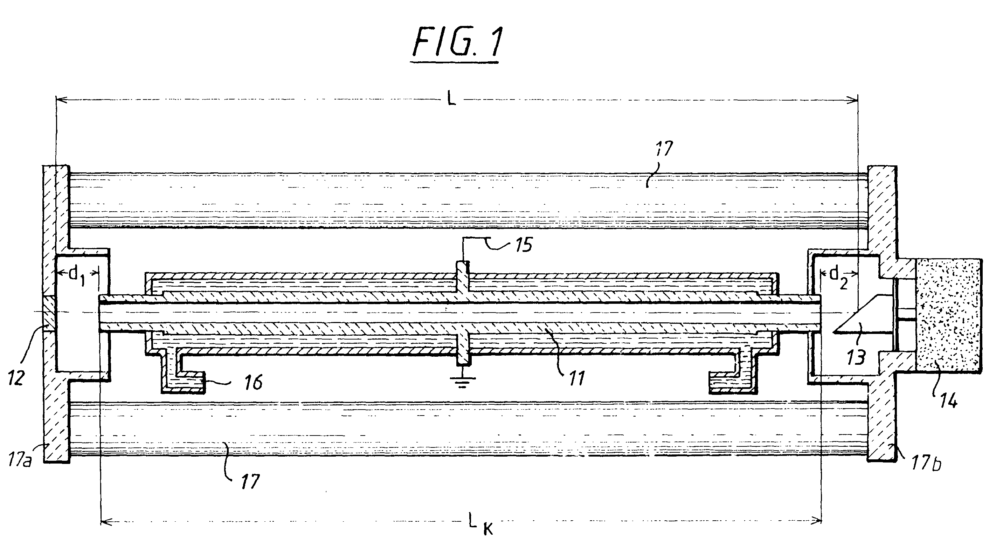

Der $CO_2$-Laser (1) nach Fig. 1 besteht aus an sich bekannten Komponenten. Ein Keramik-Wellenleiter (11) der Länge $L_K$, ein Auskoppelspiegel (12) im Abstand $d_1$ und ein Gitter (13) im Abstand $d_2$ bilden den Laser-Resonator. Mit einem piezoelektrischen Stellglied (14) ist die Resonatorlänge L verstellbar. Die Anregung erfolgt über eine HF-Leitung (15), eine Wasserkühlung (16) ist vorgesehen und das ganze ist in einem stabilen Gehäuse (17) angeordnet.

Erfindungswesentlich ist die Auswahl, Dimensionierung und Steuerung dieser Komponenten. Die Wellenlängenselektion der gewünschten Wellenlängen (hier $Lambda_1 = 10,800$ µm und $Lambda_2 = 10,784$ µm) erfolgt durch das Gitter (13) in Littrow-Anordnung. Das Gitter (13) wird derart gestaltet, im wesentlichen durch die Anzahl der Gitterlinien und damit die Auflösung, daß nur die beiden gewünschten Linien $Lambda_1$, $Lambda_2$ anschwingen können (optimale Liniendichte ca. 150 Linien/mm, Littrow-Winkel 54,1°, Reflexionseffizienz in 1. Ordnung (d.h. bei Autokollimation) ca. 98%).

Der Keramik-Wellenleiter (11) hat quadratischen Querschnitt (Fertigungsvorteile gegenüber runden) mit einer Kantenlänge von ca 2 mm und einer Länge von $L_K = 218$ mm. Für einen optimalen Betrieb entscheidend ist die Gesamtlänge des Resonators $L = d_1 + L_K + d_2$: Besitzen die beiden Linien die Frequenzen $f_1$ und $f_2$, so können bei folgender Bedingung beide Linien gleichzeitig anschwingen:

$$f_1 - f_2 = N \cdot FSR$$

mit

N =        ganze Zahl (Unterschied in der Anzahl der Halbwellen Lambda/2 von Linie 1 und 2).
FSR =      Freier Spektralbereich: c/2L (c = Lichtgeschw., L = Resonatorlänge)

Verwendet wird für den $NH_3$-Nachweis ein $CO_2$-Laser mit dem Isotop $^{13}$C, und zwar davon die Linien R(16), $Lambda_1 = 10,800$ µm: $f_1 = 27758500.36$ MHz und
R(18), $Lambda_2 = 10,784$ µm: $f_2 = 27799764.18$ MHz (vergleiche US 5 002 391).

Damit ergibt sich für N = 1 eine Gesamtlänge von 3,63 mm, d.h. alle 3,63 mm hat man identische Verhältnisse, beispielsweise:

1)   L = 3,63 mm:

672 Halbwellen Lambda$_1$   ⎫   simultane Emission von

673 Halbwellen Lambda$_2$   ⎬   Lambda$_1$ und Lambda$_2$

2)

i)   L = (3,63 + 3,63/2) mm = 5,443 mm: (genau 5,4432 mm):

1008,0 Halbwellen Lambda$_1$   ⎫   Emission nur von

1009,5 Halbwellen Lambda$_2$   ⎬   Lambda$_1$

ii)  L = 5,4432 mm + (Lambda$_1$/4 = 2,7 μm) = 5,4459 mm:

1008,5 Halbwellen Lambda$_1$   ⎫   Emission nur von

1010 Halbwellen Lambda$_2$   ⎬   Lambda$_2$

3)   L = 2 x 3,63 mm = 7,26 mm:

1344 Halbwellen Lambda$_1$   ⎫   simultane Emission von

1346 Halbwellen Lambda$_2$   ⎬   Lambda$_1$ und Lambda$_2$

Um eine zeitliche Abfolge der Laseremission auf Lambda$_1$ und Lambda$_2$ erreichen zu können, wählt man die Resonatorlänge L ähnlich dem Fall 2), nämlich

$$L = (n + 1/2) \text{ x } 3,63 \text{ mm.}$$

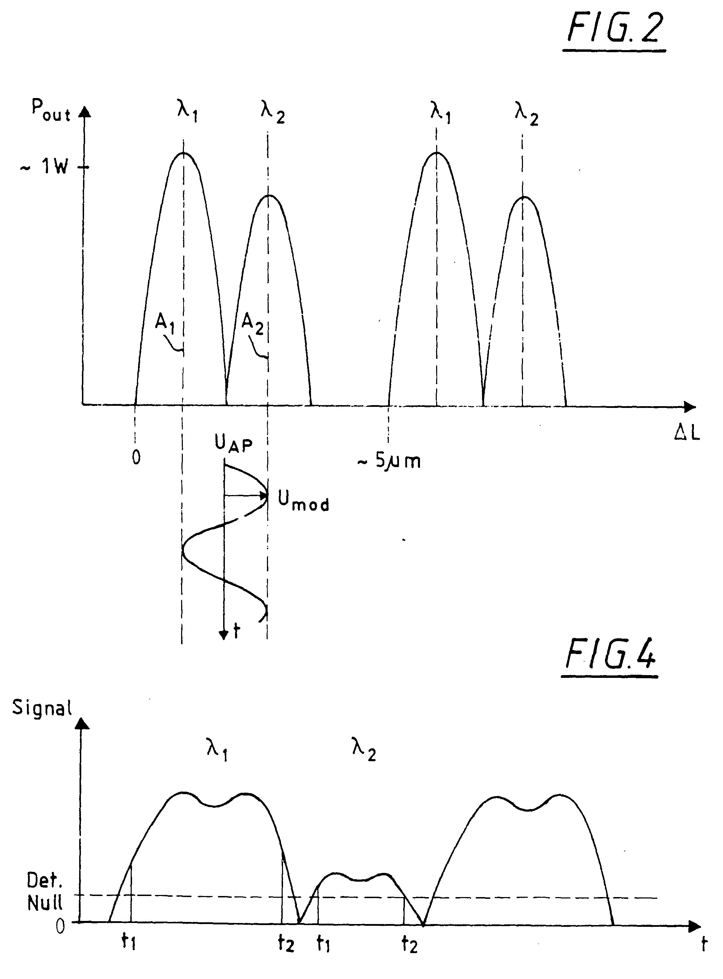

In diesem Fall ergibt sich eine maximale Trennung der beiden Linien Lambda$_1$, Lambda$_2$ in Abhängigkeit von der Resonatorlänge L (siehe Fig. 2). Im konkreten Ausführungsbeispiel ist die Resonatorlänge L auf 248,7 ± 0,2 mm (= 68,5 · 3,63 mm) eingestellt.

Die alternierende Emission erreicht man (vgl. 2)i), 2)ii)) durch sinusförmiges Verändern der Resonatorlänge L im Bereich einer viertel Wellenlänge, hier ca. ± 2,7 μm, mit dem piezoelektrischen Stellelement (14), welches das Gitter (13) in Richtung der optischen Achse des Lasers (1) linear verschiebt.

Der Krümmungsradius des Auskoppelspiegels (12) beträgt $r_{12}$ = 500 mm, der Reflexionsgrad $R_{12}$ = 90%. Dieser wird bestimmt durch die Verstärkung des Lasermediums (d.h. durch die Gas-Zusammensetzung, Gasdruck, usw.), die Verluste innerhalb des Resonators, sowie die Pumpleistung. Das Lasergas ist eine übliche Mischung aus He, $^{13}CO_2$ als laseraktives Gas, $N_2$ und Xe, der Gasdruck beträgt ca. 120 mbar und die eingekoppelte HF-Leistung liegt in der Größenordnung von 40 - 70 W. Resonatorinterne Verluste werden primär durch das Littrow-Gitter (13) (Reflexion, Streuung, Absorption) und den Wellenleiter (11) bestimmt und betragen ca. 3 - 5%. Die Laserausgangsleistungen bei Lambda$_1$ = 10,800 bzw. Lambda$_2$ = 10,784 μm betragen ca. 1,2 W.

Figur 2 zeigt eine typische Signatur des verwendeten $^{13}CO_2$-Lasers, d.h. die Ausgangsleistung $P_{out}$ als Funktion der Änderung Delta L der Resonatorlänge L.

Für den Betrieb wird eine Bias-Spannung ($U_{AP}$) an das piezoelektrische Stellelement (14) angelegt, so daß sich die Signatur im Minimum befindet. In diesem Punkt wird eine Sinusmodulation $U_{mod}$ aufgeschaltet, wodurch die Spannung am Piezoelement nach oben und unten variiert wird und der Laser (1) jeweils ins Maximum der Leistungskurve von Lambda$_1$ und anschließend ins Maximum von Lambda$_2$ gefahren wird. Abweichungen vom Maximum sind dabei zulässig. Die Bias-Spannung $U_{AP}$ wird aktiv vom programmierbaren Rechner (57) auf den Arbeitspunkt stabilisiert. Entsprechend der Modulationsfrequenz ergibt sich eine Signalfolge, die der Signaturform entspricht.

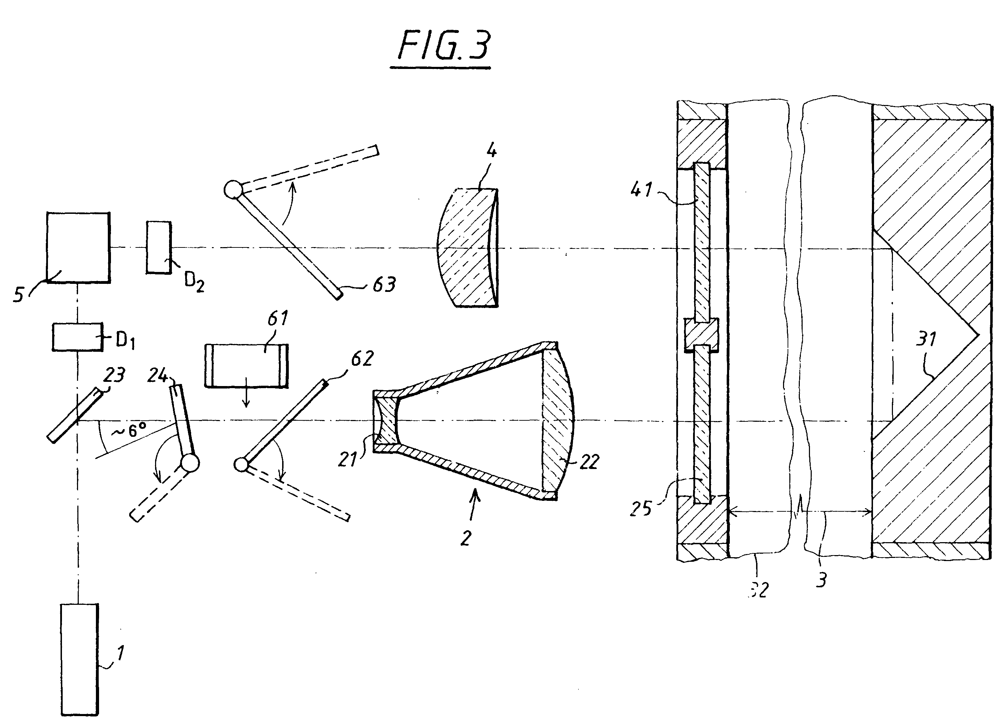

Figur 3 zeigt den optischen Aufbau eines $NH_3$-Detektors für Rauchgas in einem Rauchgaskanal (32). Von dem vom Laser (1) emittierten Laserstrahl wird an einem Strahlteiler (23) ca. 1% der Leistung zur Erfassung der momentanen Laserleistung einem Referenzdetektor (D1) zugeführt, der als pyroelektrischer Detektor ausgeführt ist. Die Sendeoptik (2) ist ein Teleskop mit zwei Linsen (21) und (22) zur Strahlaufweitung. Durch ein Abschlußfenster (25) tritt der Laserstrahl in den Rauchgaskanal (32) ein und durchquert die Meßstrecke (3). Ein Retroreflektor (31) sorgt für die Strahlumkehrung zu dem Abschlußfenster (41). In der Meßstrecke von ca. 1 m bis 20 m Länge, die im Rauchgaskanal (32) auch mehrmals gefaltet sein kann (vgl. US 5 002 391), oder auch einen beliebigen oder gar keinen Reflektor enthalten kann, wird der Laserstrahl spezifisch geschwächt und erreicht über die Empfangsoptik in Form einer Sammellinse (4) den Meßdetektor (D2), der ein MCT-Detektor ist. MCT-Detektoren sind spezielle Photowiderstände aus Quecksilber(Mercury)-Cadmium-Tellurid, die auf ca. - 60°C thermoelektrisch gekühlt werden und sehr kurze Ansprechzeiten haben.

Die Signale von Referenz- (D1) und Meßdetektor (D2) werden der Auswerteeinrichtung (5) zugeführt. Zur Messung bei sehr geringen Untergrund-Konzentrationen kann der Laserstrahl zum Schutz der Detektoren durch einen einschwenkbaren Filter (24) z.B. auf 2 Promille Leistung abgeschwächt werden. Zu Eichzwecken kann die Laserstrahlung unter Umgehung der Meßstrecke (3) durch zwei einführbare Gold-Spiegel (62, 63) direkt auf den Meßdetektor (D2) gelangen, um die Nachweise über (D1 und D2) abzugleichen ("Nullmessung"). Zusätzlich kann dann eine kalibrierte Zelle (61) mit bekanntem $NH_3$-Gehalt zum Eichen der Absorptionsstärke bei den zwei Wellenlängen $Lambda_1$, $Lambda_2$ eingeführt werden ("Referenzmessung").

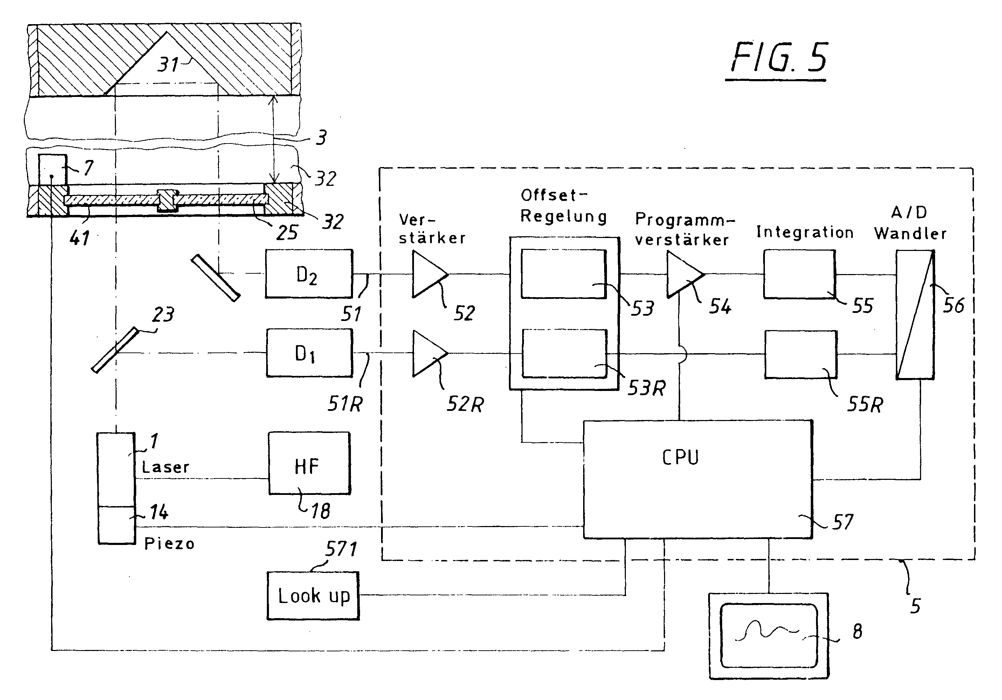

Figur 4 zeigt das so am Meßdetektor entstehende Meßsignal. Beim Übergang zwischen Laseremission bei $Lambda_1$ auf $Lambda_2$ und umgekehrt wird aufgrund der mit Fig. 2 beschriebenen Signatur jeweils der Nullpunkt erreicht. Die Zeitpunkte $t_1$ und $t_2$ zeigen jeweils die Grenzen der Integrationszeit, siehe weiter unten. Die Eindellungen in der Mitte der Signalverläufe entstehen, wenn die Modulationsspannung $U_{mod}$ die Maxima der Signatur (Fig. 2) etwas überschreitet. Eingezeichnet ist, daß die Detektoren (D1, D2) aufgrund der AC-Kopplung eine um einen Offset verschobene Null-Lage besitzen, weswegen die Offsetregelung (53, 53R) vorgesehen ist (siehe unten).

Figur 5 zeigt ein Blockschaltbild der Auswerteschaltung (5) zusammen mit den Kernelementen des optischen Aufbaues - gleiche Bezugszeichen bezeichnen gleiche Teile wie in Figur 1 und Figur 3 - und weiteren Schaltungsteilen.

Die Auswerteschaltung enthält für beide Detektoren (D1, D2) jeweils eine Wechselstromkopplung (51, 51R), einen Verstärker (52, 52R), eine Offset-Regelung (53, 53R) und eine Integrationseinrichtung (55, 55R). Dem Meßdetektor (D2) nachgeordnet ist unmittelbar vor der Integrationseinrichtung (55) eine programmiert einstellbare Verstärkungsschaltung (54), z.B. mit Schaltstufen 100-, 100-, 1000-fach. Bei sehr hohem Dynamikumfang detektorseitig kann damit die Integrationseinrichtung (55) und der nachfolgende Analog-Digital-Wandler (56), der für Meß- und Referenzsignal zugleich dient, wesentlich einfacher gestaltet werden.

Ein programmierbarer Rechner (57) wertet in bekannter Weise (vgl. US 5 002 391) die digitalisierten Signale aus dem Meßstrahlengang (3) bei $Lambda_1$ und $Lambda_2$ und vom Referenzdetektor (D1) bei $Lambda_1$ und $Lambda_2$ aus und erzeugt Konzentrationsangaben.

Eine eingegebene oder extern abgespeicherte Look-up-Tabelle (571) für die Ammoniak-Absorption erlaubt die Angabe der $NH_3$-Konzentration in beliebigen Einheiten. Mit berücksichtigt werden können dabei Daten, die von einem oder mehreren Sensoren (7) im Rauchgaskanal (32) über Druck, Temperatur oder anderes, z.B. die Kohlendioxid-Konzentration, aufgenommen werden, so daß die Absolutkonzentration von Ammoniak angegeben werden kann.

Zur Datenausgabe ist beispielsweise ein Monitor (8) vorgesehen.

Der programmierbare Rechner (57) steuert auch die Lasermodulation über die piezoelektrische Verstelleinrichtung (14) und die Offsetregelung (53, 53R) sowie die einstellbare Verstärkungsschaltung (54) und den Zeitablauf der Integration (55, 55R) und kann bedarfsgemäß für weitere Aufgaben programmiert und über Schnittstellen angeschlossen werden.

Die Laserstrahlung gelangt nach Durchlauf der Meßstrecke (3) auf den Meßdetektor (D2), ein geringer Teil der Strahlung wird durch den Strahlteiler (23) direkt auf den Referenzdetektor (D1) geleitet. Die Detektorsignale von (D1) und (D2) werden verstärkt, und zwar AC-gekoppelt, da durch den benötigten Biasstrom der Detektoren ein DC-Offsetsignal entsteht. Durch die Wechselstromkopplung (51, 51R) ergibt sich ein Schwanken der 0V-Linie in Abhängigkeit der Signalform und Signalamplitude. Da die Meß-/Referenzsignale durch Integration ausgewertet werden, ist eine Anbindung der Signale an den 0V-Pegel notwendig. Dies geschieht in der Offset-Regelung (53, 53R) mittels sample & hold: Wenn das Modulationsignal einen Nulldurchgang hat, erhält man ein Signalminimum. Die Ablage dieses Wertes zu 0V wird gesammelt und für den nächsten Meßzyklus auf das Meßsignal aufaddiert, bis im nächsten Minimum eine neue Messung stattfindet. Durch diese Maßnahme wird eine unipolare Signallage der Meßwerte erreicht, so daß eine Integration ohne Vorzeichenwechsel durchgeführt werden kann ("automatische Offset-Regelung").

Bei Inbetriebnahme des Systems wird automatisch der Arbeitspunkt ($U_{AP}$) des Lasers (1) und die Modulationsspannung $U_{mod}$ bestimmt. Da durch konstruktives Design sichergestellt ist, daß der Laser (1) nur auf den beiden gewünschten Linien $Lambda_1$, $Lambda_2$ emittiert, ist ein Spektrumanalyzer zur Kontrolle der Wellenlänge überflüssig.

Dazu wird vom Rechner (57) durchgeführt:

1. Piezoelektrisches Stellglied (14) durchfahren
2. Signatur am Referenzdetektor (D1) aufnehmen und abspeichern (Minimum und Maximum)

3. Unterschiedliche Leistungen von $Lambda_1$ und $Lambda_2$ über Flächenverhältnis von $A_1 / A_2$ (siehe Fig. 2) berücksichtigen.

4. Arbeitspunkt ($U_{AP}$) und Modulationshub $U_{mod}$ werden festgelegt.

Die bei der Wellenlänge Lambda2 gemessene Lichtschwächung bzw. Transmission, die von Ammoniak unbeeinflußt ist, kann zur Bestimmung der Rauchdichte im Meßvolumen (3) ausgenutzt werden. Genau ist das möglich, wenn die Komponenten des Abgas/Rauch-Stroms an sich und der Extinktionskoeffizient des Rauchs bekannt sind. Die verwendete große Wellenlänge im Infraroten ist dabei vorteilhaft, da durch die geringe Streuung relativ große Meßlängen und Rauchdichten erfaßt werden können. Besonderer Vorteil ist, daß diese zusätzliche Messung nur durch entsprechende Programmierung des Rechners (57) erhalten wird.

Die Beschreibung der Erfindung an einem konkreten Beispiel bedeutet keine Beschränkung, Abwandlungen sind zahlreich möglich. Wichtig ist, daß die erfindungsgemäße Ausführung es ermöglicht, ohne Chopper oder ähnliche zusätzliche Lichtmodulatoren in der Stoffnachweisanordnung auszukommen, da die Signatur des Lasers (1) für einen zuverlässigen Nulldurchgang der Laserintensität sorgt. Die Sinusmodulation des Laserlichtes kann von den Detektoren (D1) und (D2) und der Auswerteschaltung (5) im Unterschied zu einer Rechteckmodulation formtreu, ohne Verluste bei der Fourierzerlegung bzw. beim Frequenzgang, verarbeitet werden. Damit ist die Integration der Signale und somit die hohe Ausnutzung der Laserenergie für die Signalgewinnung möglich. Rechteckmodulation mit Sample-and-hold-Signalverarbeitung muß dagegen einen Großteil des Laserpulses unberücksichtigt lassen. Eine Referenzzelle mit kalibrierten $NH_3$-Gehalt wird ebenfalls nicht ständig mit einem eigenen Detektor ausgemessen. Es genügt, diese gelegentlich zu Eichzwecken einzuführen und dann den vorhandenen Meßdetektor (D2) zu benutzen.

## Patentansprüche

1. Gaslaser, der durch sinusförmige Verschiebung eines Resonatorspiegels oder eines Gitters (13) bei zwei verschiedenen Wellenlängen ($Lambda_1$, $Lambda_2$) emittiert, dadurch gekennzeichnet, daß durch geeignete Dimensionierung eines Gitters (13) hinsichtlich Gitterstruktur und Winkellage, des Auskoppelgrads des Auskoppelspiegels (12), der Resonatorlänge (L) und der Gaszusammensetzung der Laser in zwei nebeneinanderliegenden Intervallen der Resonatorlänge (L) bei den zwei gewünschten Wellenlängen ($Lambda_1$ und $Lambda_2$) emittiert und am Übergang zwischen den zwei Intervallen die Laseremission unterbunden ist, und daß die sinusförmige Modulation der Resonatorlänge (L) im wesentlichen symmetrisch um diesen Übergang liegt.

2. Gaslaser nach Anspruch 1, dadurch gekennzeichnet, daß er ein $CO_2$-Laser mit einem Gitter in Littrow-Anordnung ist.

3. Gaslaser nach Anspruch 1 oder Anspruch 2, dadurch gekennzeichnet, daß die Resonatorlänge (L) nach $L = (n + 1/2) \cdot L_0$ bestimmt ist, wobei n ganzzahlig ist und $L_0$ die kleinste Länge ist, bei der zugleich für beide Wellenlängen ($Lambda_1$, $Lambda_2$), bei welchen der Laser (1) emittiert, Resonanz gegeben ist.

4. Gaslaser nach Anspruch 2 oder 3, dadurch gekennzeichnet, daß er das Isotop $^{13}C$ enthält und bei den Linien R (16) mit der Wellenlänge $Lambda_1 = 10{,}800$ µm und R (18) mit $Lambda_2 = 10{,}784$ µm emittiert.

5. Gaslaser nach mindestens einem der Ansprüche 1 - 4, dadurch gekennzeichnet, daß ein piezoelektrisches Stellelement (14) zur linearen Verschiebung des Gitters (13) in Richtung der Resonatorachse vorgesehen ist und damit die emittierte Wellenlänge ($Lambda_1$, $Lambda_2$) bestimmt wird.

6. Verwendung eines Gaslasers (1) nach mindestens einem der Ansprüche 1 - 5 in einer Anordnung zum Stoffnachweis durch unterschiedliche Absorption von Licht zweier Wellenlängen ($Lambda_1$, $Lambda_2$).

7. Verwendung eines Gaslasers (1) nach Anspruch 6 gekennzeichnet durch den Nachweis von Spurengas in Gas, insbesondere von $NH_3$ in Rauchgas.

8. Anordnung zum Stoffnachweis mit einer 2-Wellenlängen-Laserlichtquelle (1),

- einer Modulationseinrichtung (14) zum Verstellen der Resonatorlänge (L) und einer Referenz-Detektionseinrichtung (D1) zur Erfassung der momentanen Laserleistung,
- einer Sendeoptik (2)
- einer sich anschließenden Meßstrecke (3), welche den nachzuweisenden Stoff enthält,

- einer Empfangsoptik (4) für das Laserlicht, das die Meßstrecke passiert hat, und einem sich anschließenden Meßdetektor (D2)
- und einer Auswerteeinrichtung (5), welche das Verhältnis der Lichtabsorption in der Meßstrecke (3) bei den zwei Wellenlängen (Lambda$_1$, Lambda$_2$) und daraus die Konzentration des nachzuweisenden Stoffes in der Meßstrecke (3) bestimmt, dadurch gekennzeichnet, daß die 2-Wellenlängen-Laserlichtquelle (1) ein Laser nach einem oder mehreren der Ansprüche 1 - 5 ist.

9. Anordnung nach Anspruch 8, dadurch gekennzeichnet, daß die Meßstrecke (3) offen ist und einen Reflektor (31) enthält.

10. Eine Anordnung nach Anspruch 8 oder Anspruch 9, dadurch gekennzeichnet, daß zum Zweck einer Referenzmessung eine Zelle (61) mit bekannter Konzentration des nachzuweisenden Stoffs in den Strahlengang eingebracht werden kann.

11. Anordnung nach Anspruch 9 oder 10, dadurch gekennzeichnet, daß die Meßstrecke (3) in einem Rauchgaskanal (32) liegt und der nachzuweisende Stoff NH$_3$ ist.

12. Anordnung nach mindestens einem der Ansprüche 8 - 11, dadurch gekennzeichnet, daß Mittel (24) zur Einstellung der in die Meßstrecke (3) eingestrahlten Laserleistung zur Anpassung an die Konzentration des nachzuweisenden Stoffs oder des Untergrunds vorhanden sind.

13. Anordnung nach mindestens einem der Ansprüche 8 - 12, dadurch gekennzeichnet, daß die Anordnung chopperlos aufgebaut ist mit einem dauernd lichtdurchgängigen Strahlungsweg von der Laserlichtquelle (1) zum Meßdetektor.

14. Anordnung nach mindestens einem der Ansprüche 8 - 13, dadurch gekennzeichnet, daß höchstens zwei Detektoren für das Laserlicht vorhanden sind.

15. Anordnung nach mindestens einem der Ansprüche 8 - 14, dadurch gekennzeichnet, daß der Meßdetektor (D2) ein MCT-Detektor ist.

16. Anordnung nach mindestens einem der Ansprüche 8 - 15, dadurch gekennzeichnet, daß die Auswerteeinrichtung (5) eine Wechselstromkopplung (51, 51R) des Referenz-(D1) und des Meßdetektors (D2) eine Verstärkungsschaltung (52, 52R, 54) zur Verstärkung der Signale des Referenz-(D1) und Meßdetektors (D2) und eine nachgeschaltete Integrationseinrichtung (55, 55R) enthält, sowie einen anschließenden Analog-Digital-Wandler (56) und einen programmierbaren Rechner (57).

17. Anordnung nach Anspruch 16, dadurch gekennzeichnet, daß die Integrationseinrichtung (55, 55R) so ausgebildet ist, daß sie das vom Meßdetektor (D2) aufgenommene Signal jeweils über den überwiegenden Teil (t$_1$ - t$_2$) des Zeitintervalls, in dem der Gaslaser (1) bei einer Wellenlänge (Lambda$_1$, Lambda$_2$) emittiert, aufintegriert.

18. Anordnung nach Anspruch 16 oder 17, dadurch gekennzeichnet, daß sie so ausgebildet ist, daß die Verstärkungsschaltung (54) in ihrem Verstärkungsfaktor abhängig vom Signal des Meßdetektors (D2) automatisch verstellt wird.

19. Anordnung nach mindestens einem der Ansprüche 8 - 18, dadurch gekennzeichnet, daß die Meßstrecke (3) in einem Rauchgaskanal (32) liegt und der nachzuweisende Stoff NH$_3$ ist, und daß die Auswerteeinrichtung (5) so ausgebildet ist, daß aus den bei der Wellenlänge (Lambda$_2$), bei der Ammoniak nicht absorbiert, gewonnenen Absorptionsdaten zusätzlich ein Meßwert für die Rauchdichte in der Meßstrecke (3) bestimmt und ausgegeben wird.

**Claims**

1. Gas laser which emits at two different wavelengths (lambda$_1$, lambda$_2$) by sinusoidal displacement of a resonator mirror or a grating (13), characterized in that owing to suitable dimensioning of a grating (13) with regard to grating structure and angular position, the outcoupling factor of the outcoupling mirror (12), the resonator length (L) and the gas composition, the laser emits in two juxtaposed intervals of the resonator length (L) at the two desired wavelengths (lambda$_1$ and lambda$_2$), and the laser emission is suppressed at the transition between the two intervals, and in that the sinusoidal modulation of the resonator length (L) is situated essentially symmetrically about this transition.

2. Gas laser according to Claim 1, characterized in that it is a $CO_2$ laser with a grating in a Littrow arrangement.

3. Gas laser according to Claim 1 or Claim 2, characterized in that the resonator length (L) is determined in accordance with $L = (n + 1/2) \cdot L_0$, n being an integer and $L_0$ being the smallest length for which resonance occurs simultaneously for both wavelengths ($\text{lambda}_1$, $\text{lambda}_2$) at which the laser (1) emits.

4. Gas laser according to Claim 2 or 3, characterized in that it contains the isotope $^{13}C$ and emits at the lines R (16) with the wavelength $\text{lambda}_1$ = 10.800 μm and R (18) with $\text{lambda}_2$ = 10.784 μm.

5. Gas laser according to at least one of Claims 1-4, characterized in that a piezoelectric control element (14) is provided for linear displacement of the grating (13) in the direction of the resonator axis, and the emitted wavelength ($\text{lambda}_1$, $\text{lambda}_2$) is determined thereby.

6. Use of a gas laser (1) according to at least one of Claims 1-5 in an arrangement for detecting material by means of different absorption of light of two wavelengths ($\text{lambda}_1$, $\text{lambda}_2$).

7. Use of a gas laser (1) according to Claim 6, characterized by the detection of trace gas in gas, in particular of $NH_3$ in flue gas.

8. Arrangement for detecting material, having a two-wavelength laser light source (1),

   - a modulation device (14) for adjusting the resonator length (L), and a reference detection device (D1) for detecting the instantaneous laser power,
   - an optical transmitting system (2),
   - an adjoining measurement section (3) which contains the material to be detected,
   - an optical receiving system (4) for the laser light which has passed the measurement section, and an adjoining measuring detector (D2),
   - and an evaluation device (5) which determines the ratio of the light absorption in the measurement section (3) at the two wavelengths ($\text{lambda}_1$, $\text{lambda}_2$) and determines therefrom the concentration of the material to be detected in the measurement section (3), characterized in that the two-wavelength laser light source (1) is a laser according to one of Claims 1-5.

9. Arrangement according to Claim 8, characterized in that the measurement section (3) is open and contains a reflector (31).

10. Arrangement according to Claim 8 or Claim 9, characterized in that a cell (61) having a known concentration of the material to be detected can be brought into the beam path for the purpose of a reference measurement.

11. Arrangement according to Claim 9 or 10, characterized in that the measurement section (3) is situated in a flue gas duct (32), and the material to be detected is NH3.

12. Arrangement according to at least one of Claims 8-11, characterized in that means (24) are present for setting the laser power radiated into the measurement section (3) for the purpose of adaptation to the concentration of the material to be detected, or that of the background.

13. Arrangement according to at least one of Claims 8-12, characterized in that the arrangement is designed without a chopper and with a beam path which is permanently light-transmitting from the laser light source (1) to the measuring detector.

14. Arrangement according to at least one of Claims 8-13, characterized in that at most two detectors are present for the laser light.

15. Arrangement according to at least one of Claims 8-14, characterized in that the measuring detector (D2) is an MCT detector.

16. Arrangement according to at least one of Claims 8-15, characterized in that the evaluation device (5) includes an AC coupling (51, 51R) of the reference detector (D1) and the measuring detector (D2), an amplifier circuit (52, 52R, 54) for amplifying the signals of the reference detector (D1) and measuring detector (D2) and a downstream integration device (55, 55R), as well as an adjoining analogue-to-digital converter (56) and a programmable computer (57).

17. Arrangement according to Claim 16, characterized in that the integration device (55, 55R) is designed such that in each case it integrates the signal picked up by the measuring detector (D2) over the predominant portion ($t_1$-$t_2$) of the time interval in which the gas laser (1) emits at one wavelength ($lambda_1$, $lambda_2$).

18. Arrangement according to Claim 15 or 17, characterized in that it is designed such that the gain of the amplifying circuit (54) is automatically adjusted as a function of the signal from the measuring detector (D2).

19. Arrangement according to at least one of Claims 8-18, characterized in that the measurement section (3) is situated in a flue gas duct (32) and the material to be detected is NH3, and in that the evaluation device (5) is designed such that absorption data obtained at the wavelength (lambda2) at which ammonia does not absorb are used additionally to determine and output a measured value for the smoke density in the measurement section (3).

## Revendications

1. Laser à gaz qui émet sur deux longueurs d'onde différentes ($Lambda_1$, $Lambda_2$) par décalage sinusoïdal d'un miroir de cavité ou d'une grille (13), caractérisé en ce que le laser émet en deux intervalles juxtaposés de la longueur de la cavité (L) aux deux longueurs d'onde ($Lambda_1$, $Lambda_2$) voulues par un dimensionnement approprié d'une grille (13) du point de vue de la structure de la grille et de la position angulaire, du degré de déclenchement du miroir de sortie (12), de la longueur de la cavité (L) et de la composition du gaz et que l'émission du laser est interrompue lors de la transition entre les deux intervalles, et en ce que la modulation sinusoïdale de la longueur de la cavité (L) est essentiellement symétrique à cette transition.

2. Laser à gaz selon la revendication 1, caractérisé en ce qu'il s'agit d'un laser $CO_2$ avec une grille à disposition en Littrow.

3. Laser à gaz selon la revendication 1 ou la revendication 2, caractérisé en ce que la longueur de la cavité (L) est déterminée selon L = (n + ½) .$L_0$, où n est un nombre entier et $L_0$ la plus petite longueur pour laquelle il existe, en même temps, une résonance pour les deux longueurs d'onde ($Lambda_1$, $Lambda_2$) auxquelles émet le laser (1).

4. Laser à gaz selon la revendication 2 ou 3, caractérisé en ce qu'il contient l'isotope $^{13}$C et qu'il émet à la longueur d'onde $Lambda_1$ = 10,800 µm dans le cas des lignes R (16) et à $Lambda_2$ = 10,784 µm dans le cas des lignes R (18).

5. Laser à gaz selon au moins l'une des revendications 1 à 4, caractérisé en ce qu'un élément de réglage piézoélectrique (14) est prévu pour le décalage linéaire de la grille (13) en direction de l'axe de la cavité et que la longueur d'onde émise ($Lambda_1$, $Lambda_2$) est ainsi déterminée.

6. Utilisation d'un laser à gaz (1) selon au moins l'une des revendications 1 à 5 dans un arrangement pour détecter une substance par différence d'absorption de lumière de deux longueurs d'onde ($Lambda_1$, $Lambda_2$).

7. Utilisation d'un laser à gaz (1) selon la revendication 6, caractérisée par la détection de traces de gaz dans un gaz, notamment de NH3 dans le gaz de combustion.

8. Arrangement pour détecter une substance avec une source de lumière laser à 2 longueurs d'onde (1),

   * un dispositif de modulation (14) pour régler la longueur de la cavité (L) et un dispositif de détection de référence (D1) pour détecter la puissance momentanée du laser,
   * une optique d'émission (2),
   * une section de mesure (3) qui vient se raccorder et qui contient la substance à détecter,
   * une optique de réception (4) de la lumière laser qui a traversé la section de mesure et un détecteur de mesure (D2) qui vient se raccorder
   * et un dispositif d'analyse (5) qui détermine la proportion d'absorption de lumière dans la section de mesure (3) aux deux longueurs d'onde ($Lambda_1$, $Lambda_2$), et de là la concentration de la substance à détecter dans la section de mesure (3), caractérisé en ce que la source de lumière laser à 2 longueurs d'onde (1) est un laser selon l'une des revendications 1 à 5.

9.  Arrangement selon la revendication 8, caractérisé en ce que la section de mesure (3) est ouverte et qu'elle contient un réflecteur (31).

10. Arrangement selon la revendication 8 ou la revendication 9, caractérisé en ce qu'une cellule (61) contenant une concentration connue de la substance à détecter peut être insérée dans le trajet des rayons afin de réaliser une mesure de référence.

11. Arrangement selon la revendication 9 ou 10, caractérisé en ce que la section de mesure (3) se trouve dans un conduit de gaz de combustion (32) et que la substance à détecter est du $NH_3$.

12. Arrangement selon au moins l'une des revendications 8 à 11, caractérisé en ce qu'il existe des moyens (24) pour régler la puissance du laser rayonnée dans la section de mesure (3) afin de l'adapter à la concentration de la substance à détecter ou du fond.

13. Arrangement selon au moins l'une des revendications 8 à 12, caractérisé en ce que l'arrangement est construit sans vibrateur avec un chemin de rayonnement constamment transparent de la source de lumière laser (1) jusqu'au détecteur de mesure.

14. Arrangement selon au moins l'une des revendications 8 à 13, caractérisé en ce qu'il existe au maximum deux détecteurs pour la lumière laser.

15. Arrangement selon au moins l'une des revendications 8 à 14, caractérisé en ce que le détecteur de mesure (D2) est un détecteur MCT.

16. Arrangement selon au moins l'une des revendications 8 à 15, caractérisé en ce que le dispositif d'analyse (5) comprend un couplage pour courant alternatif (51, 51R), des détecteurs de référence (D1) et de mesure (D2), un circuit amplificateur (52, 52R, 54) pour amplifier les signaux des détecteurs de référence (D1) et de mesure (D2) et un dispositif d'intégration (55, 55R) branché en aval ainsi qu'un convertisseur analogique/numérique (56) qui y est branché et un calculateur programmable (57).

17. Arrangement selon la revendication 16, caractérisé en ce que le dispositif d'intégration (55, 55R) est configuré de telle manière à intégrer le signal en provenance du détecteur de mesure (D2) à chaque fois par le biais de la partie prépondérante ($t_1$- $t_2$) de l'intervalle de temps pendant lequel le laser à gaz (1) émet à l'une des longueurs d'onde ($Lambda_1$, $Lambda_2$).

18. Arrangement selon la revendication 15 ou 17, caractérisé en ce qu'il est configuré de telle manière que le facteur d'amplification du circuit d'amplification (54) est réglé automatiquement en fonction du signal du détecteur de mesure (D2).

19. Arrangement selon au moins l'une des revendications 8 à 18, caractérisé en ce que la section de mesure (3) se trouve dans un conduit de gaz de combustion (32) et que la substance à détecter est du NH3 et que le dispositif d'analyse (5) est configuré de telle manière que les données d'absorption acquises à la longueur d'onde (Lambda2), que n'absorbe pas l'ammoniac, permettent en plus de déterminer et de produire une valeur mesurée pour la densité de la fumée dans la section de mesure (3).

FIG.1

EP 0 677 733 B1

_FIG.2_

_FIG.4_

FIG.3

FIG.5

*31*

*3*

*7*

*32*

*32*

*41*

*25*

*23*

| Ver-stärker | Offset-Regelung | Programm-verstärker | Integration | A/D Wandler |

D₂  *51*  *52*  *53*  *54*  *55*  *56*

D₁  *51R*  *52R*  *53R*  *55R*

*1* Laser  HF  *18*

*14* Piezo

CPU  *57*

*571* Look up

*8*

*5*

EP 0 677 733 B1